Europäisches Patentamt

European Patent Office

Office européen des brevets

(11) Numéro de publication : **0 519 783 A1**

(19)

## (12) DEMANDE DE BREVET EUROPEEN

(21) Numéro de dépôt : **92401595.1**

(22) Date de dépôt : **10.06.92**

(51) Int. Cl.⁵ : **G01N 33/00**, G01N 1/22, G01N 30/00, G01N 19/10, G01D 18/00

(30) Priorité : **12.06.91 FR 9107139**

(43) Date de publication de la demande : **23.12.92 Bulletin 92/52**

(84) Etats contractants désignés : **DE FR GB IT**

(71) Demandeur : **L'AIR LIQUIDE, SOCIETE ANONYME POUR L'ETUDE ET L'EXPLOITATION DES PROCEDES GEORGES CLAUDE 75, Quai d'Orsay F-75321 Paris Cédex 07 (FR)**

(72) Inventeur : **Scotto d'Appollonia, Sylvain 23 rue Maurice Ravel F-78280 Guyancourt (FR)** Inventeur : **Molozay, Maurice 1 allée des Charpentiers F-78320 Le Mesnil Saint Denis (FR)**

(74) Mandataire : **Le Moenner, Gabriel et al L'AIR LIQUIDE, Société Anonyme pour l'étude et l'exploitation des procédés Georges Claude 75, Quai d'Orsay F-75321 Paris Cédex 07 (FR)**

(54) **Procédé et dispositif de fourniture de gaz à un analyseur à haute sensibilité.**

(57)     Le procédé consiste à diviser un flux primaire $(Q)$ d'un gaz vecteur en une série de flux à débits déterminés $(q_1-q_{10})$, d'introduire dans un premier de ces flux $(q_1)$ une quantité déterminée du gaz à analyser $(A)$ pour obtenir un premier mélange $(M_1)$, de diluer ce premier mélange $(M_1)$ avec un flux de gaz vecteur pour constituer un deuxième mélange $(M_2)$, de diluer ce deuxième mélange avec un flux $(q_5)$ du gaz vecteur pour constituer un troisième mélange $(M_3)$, et ainsi de suite, ainsi que de fournir un gaz étalon $(U_5)$ à très faible teneur en deuxième gaz, de façon à offrir en plusieurs sorties, utilisables en parallèle, des débits contrôlés de différents mélanges couvrant différentes plages depuis de très faibles teneurs jusqu'à des teneurs plus élevées en gaz à analyser.

Application notamment à l'étalonnage d'hygromètres haute sensibilité.

FIG.1

EP 0 519 783 A1

La présente invention concerne un procédé de fourniture, à au moins un analyseur à haute sensibilité, de faibles quantités d'un premier gaz contenues dans un second gaz, comprenant les étapes de diviser un flux primaire du second gaz en un premier flux et un deuxième flux supérieur au premier flux, d'introduire dans le premier flux une quantité déterminée du premier gaz pour constituer un premier mélange, et de combiner au moins une partie du deuxième flux avec le premier mélange pour constituer un deuxième mélange disponible en une première sortie d'utilisation pour fourniture à l'analyseur.

Les techniques modernes requièrent de plus en plus des capacités à détecter de très faibles quantités, pouvant descendre jusqu'au ppb (partie par milliard) d'un premier gaz dans un second gaz. Les analyseurs à très haute sensibilité prévus à cet effet présentent des problèmes d'étalonnage difficiles à résoudre, en particulier pour les hygromètres. Les procédés connus, du type défini ci-dessus, mettant en oeuvre une dilution du premier mélange avec le deuxième flux ne permettent de couvrir, à la sortie d'utilisation, qu'une plage limitée, le passage à une autre plage nécessitant une modification des moyens d'injection dans le premier flux de la quantité déterminée du premier gaz.

La présente invention a pour objet de proposer un procédé permettant, de façon fiable, reproductible et modulable, de couvrir, avec un système unique, d'utilisation souple et peu onéreuse, une vaste plage d'étalonnage en sorties en plusieurs sorties, pouvant descendre jusqu'à des niveaux très bas, inférieurs à 0,01 ppm (parties par million) permettant une mise rapide en régime de fonctionnement et garantissant une stabilité des taux de dilution dans les différents mélanges de sortie.

Pour ce faire, selon une caractéristique de l'invention, le procédé comprend les étapes de diviser le deuxième flux en un troisième flux, pour combinaison avec le premier mélange, et un quatrième flux, supérieur au troisième flux, de prélever une partie du deuxième mélange avant fourniture à la première sortie d'utilisation, et de combiner au moins une partie du quatrième flux avec la partie prélevée du deuxième mélange pour constituer un troisième mélange disponible en une deuxième sortie d'utilisation pour fourniture à un analyseur , le troisième mélange ayant ainsi une teneur déterminée en premier gaz inférieure à celle du premier mélange disponible à la première sortie d'utilisation.

De façon plus spécifique, le procédé comprend en outre les étapes de diviser le quatrième flux en un cinquième flux pour combinaison avec la partie prélevée du deuxième mélange, et un sixième flux supérieur au cinquième flux, de prélever une partie du troisième mélange avant fourniture à la deuxième sortie, de combiner au moins une partie du sixième flux avec la partie du troisième mélange pour constituer un quatrième mélange disponible à une troisième sortie, typiquement de diviser le sixième flux en un septième flux, pour combinaison avec la partie prélevée du troisième mélange, et un huitième flux supérieur au septième flux, de prélever une partie du quatrième mélange avant fourniture à la troisième sortie, de combiner au moins une partie du septième flux avec la partie prélevée du quatrième mélange pour constituer un cinquième mélange disponible à une quatrième sortie, et avantageusement de diviser le huitième flux en un neuvième flux, pour combinaison avec la partie prélevée du quatrième mélange, et un dixième flux, de prélever une partie du quatrième mélange avant fourniture à la troisième sortie, de faire passer le dixième flux dans un séparateur, typiquement un piège cryogénique pour fourniture d'un mélange étalon, via une cinquième sortie, avantageusement après combinaison avec une partie de la partie prélevée du quatrième mélange.

Avec un tel agencement, à partir d'un flux primaire divisé plusieurs fois et de l'introduction d'une quantité déterminée du premier gaz dans le deuxième gaz en un poste unique, on obtient aux différentes sorties d'utilisation des plages modulables et échelonnées par exemple entre 1000 ppm et 0,01 ppm ou moins, deux sorties différentes pouvant être utilisées en parallèle pour la calibration zéro ou l'étalonnage de deux analyseurs.

La présente invention a également pour objet un dispositif de fourniture de gaz à au moins un analyseur à très haute sensibilité pour la mise en oeuvre du procédé ci-dessus, du type comprenant une source de deuxième gaz alimentant une première ligne comportant un moyen d'introduction du deuxième gaz, et une deuxième ligne reliée à la première ligne en aval du moyen d'introduction et se terminant par un premier tronçon de sortie connectable à l'analyseur, caractérisé en ce que la deuxième ligne se divise en une troisième ligne reliée à une première dérivation provenant du premier tronçon de sortie et se terminant en un second tronçon de sortie connectable à l'analyseur.

De façon plus spécifique, la deuxième ligne se divise une quatrième ligne reliée à une deuxième dérivation provenant du deuxième tronçon de sortie et se terminant en un troisième tronçon de sortie, la deuxième ligne se divisant typiquement en une cinquième ligne reliée à une troisième dérivation du troisième tronçon de sortie et se terminant en un quatrième tronçon de sortie connectable à l'analyseur, la deuxième ligne se divisant avantageusement en une sixième ligne, traversant un piège cryogénique, reliée à la troisième dérivation et se terminant en un cinquième tronçon de sortie connectable à l'analyseur.

De façon plus spécifique, pour l'application à l'étalonnage d'hygromètres, le premier gaz étant de la vapeur d'eau, le moyen d'introduction est constitué d'un saturateur thermostaté à plan d'eau, avantageusement à double enveloppe et à pression contrôlée.

D'autres caractéristiques et avantages de la présente invention ressortiront de la description suivante d'un mode de réalisation de l'invention, donné à titre illustratif mais nullement limitatif, faite en relation avec les dessins annexés, sur lesquels :

- la figure 1 est une représentation schématique d'un dispositif de fourniture de gaz selon l'invention ; et

- la figure 2 est une vue schématique d'une installation selon la figure 1 pour le calibrage et l'étalonnage d'hygromètres.

Dans la description qui va suivre et sur les dessins, les éléments identiques ou analogues portent les mêmes chiffres de référence, éventuellement indicés.

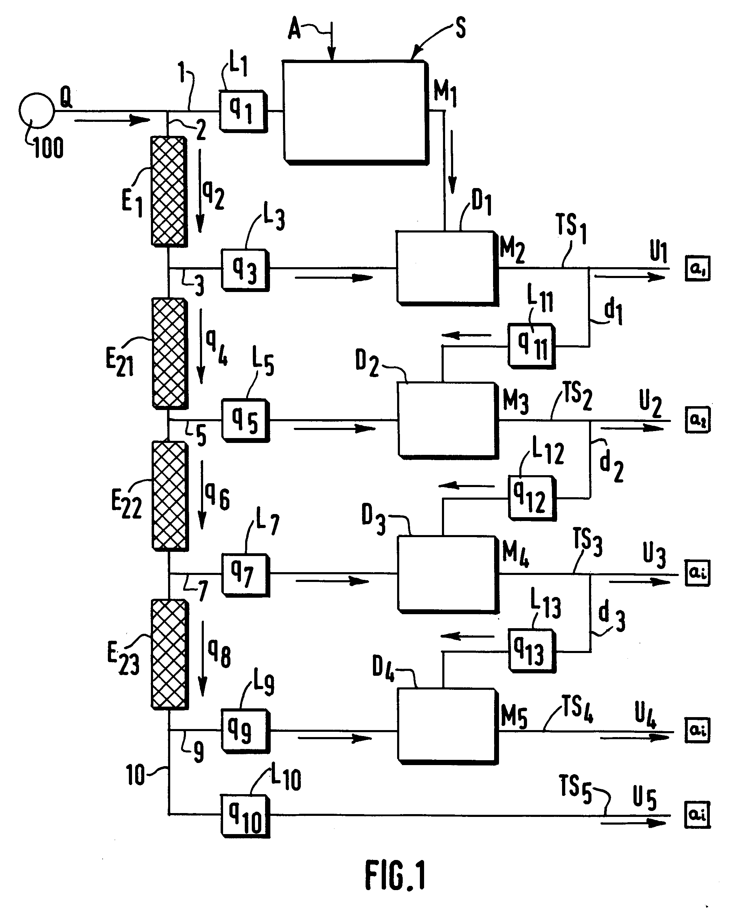

Sur le schéma de la figure 1, un flux Q d'un gaz vecteur, typiquement un gaz inerte tel que l'azote ou l'argon, provenant d'une source 100 alimente une première ligne 1, comportant un poste S d'introduction d'une faible quantité déterminée d'un gaz d'analyse A dans le gaz vecteur parcourant la première ligne 1 et fournissant en sortie un premier mélange de gaz M1. La source de gaz vecteur 100 alimente également une deuxième ligne 2, pourvue d'un premier dispositif épurateur $E_1$ en aval duquel elle se divise en une troisième ligne 3 se raccordant, en un premier poste de dilution $D_1$, à la première ligne 1 en aval du dispositif d'introduction S du gaz d'analyse A pour se terminer par un premier tronçon de sortie $T_1$ connectable, en $U_1$ à un analyseur. La deuxième ligne 2 se prolonge, en aval de l'épurateur $E_1$ traverse un épurateur $E_{21}$ et se divise en une cinquième ligne 5 se raccordant, en un second poste de dilution $D_2$, à une première dérivation $d_1$ provenant du premier tronçon de sortie $S_1$ pour se terminer en un second tronçon de sortie $TS_2$ connectable, en $U_2$, à un analyseur. La deuxième ligne 2 se prolonge en traversant un épurateur $E_{22}$ et se divise en une septième ligne 7 se raccordant, en un troisième poste de dilution $D_3$, à une seconde dérivation $d_2$ provenant du second tronçon de sortie $TS_2$, pour se terminer en un troisième tronçon de sortie $TS_3$ connectable, en $U_3$, à un analyseur. La deuxième ligne se prolonge en traversant un épurateur $E_{23}$ et se divise en une neuvième ligne 9 et une dixième ligne 10. La neuvième ligne 9 se raccorde, en un quatrième poste de dilution $D_4$, à une troisième dérivation $d_3$ provenant du troisième tronçon $TS_3$ pour se terminer en un quatrième tronçon de sortie $TS_4$ connectable, en $U_4$, à un analyseur. La dixième ligne 10 se termine en un cinquième tronçon de sortie $TS_5$ connectable, en $U_5$, à un analyseur. Les débits $q_1$, $q_3$, $q_5$, $q_7$, $q_9$ et $q_{10}$ dans les lignes 1, 3, 5, 7, 9 et 10 sont fixés par des moyens limiteurs de débit $L_1$, $L_3$, $L_5$, $L_7$, $L_9$ et $L_{10}$, respectivement. Avantageusement, les débits $q_{11}$, $q_{12}$, $q_{13}$ dans les dérivations $d_1$, $d_2$, $d_3$ sont fixés par des moyens limiteurs de débit $L_{11}$, $L_{12}$, $L_{13}$, respectivement.

Les épurateurs $E_1$ et $E_{2i}$ sont des épurateurs du type à adsorbant à zéolithe et/ou charbon actif pour épurer le gaz vecteur de ses éventuels contaminants, vapeur d'eau, méthane ou $CO_2$. Les différentes tubulures sont réalisées en acier inoxydable électropolies intérieurement.

On comprendra qu'avec un tel agencement, à partir d'un flux primaire de gaz vecteur Q et d'un premier mélange déterminé $M_1$ du gaz vecteur et d'une faible quantité du gaz à analyser A, on obtient, à la première sortie $U_1$ un deuxième mélange $M_2$ des deux gaz avec une première teneur réduite en gaz à analyser, en une deuxième sortie $U_2$, un troisième mélange de gaz $M_3$ avec une seconde teneur plus diluée que la première teneur en gaz à analyser, et ainsi de suite dans les sorties $U_3$ à $U_5$, cette dernière sortie fournissant un gaz étalon ou gaz zéro correspondant au gaz vecteur contenant une quantité infime du gaz à analyser, typiquement inférieure à 0,005 ppm, chaque dilution, aux postes $D_1$ à $D_4$ s'effectuant avec le même gaz vecteur sensiblement pur et exempt d'impuretés, notamment du type de celles du gaz à analyser A. Les différents moyens limiteurs de débit $L_1$ à $L_{13}$ permettent de garantir des débits déterminés constants aux différentes sorties $U_1$ à $U_5$ quelles que soient les utilisations faites de ces différentes sorties. De ce fait, on peut utiliser en parallèle deux ou plus de ces sorties, qui offrent différentes gammes, couvrant une large plage, de teneurs en gaz à analyser, pour effectuer l'étalonnage ou la calibration de plusieurs analyseurs simultanément.

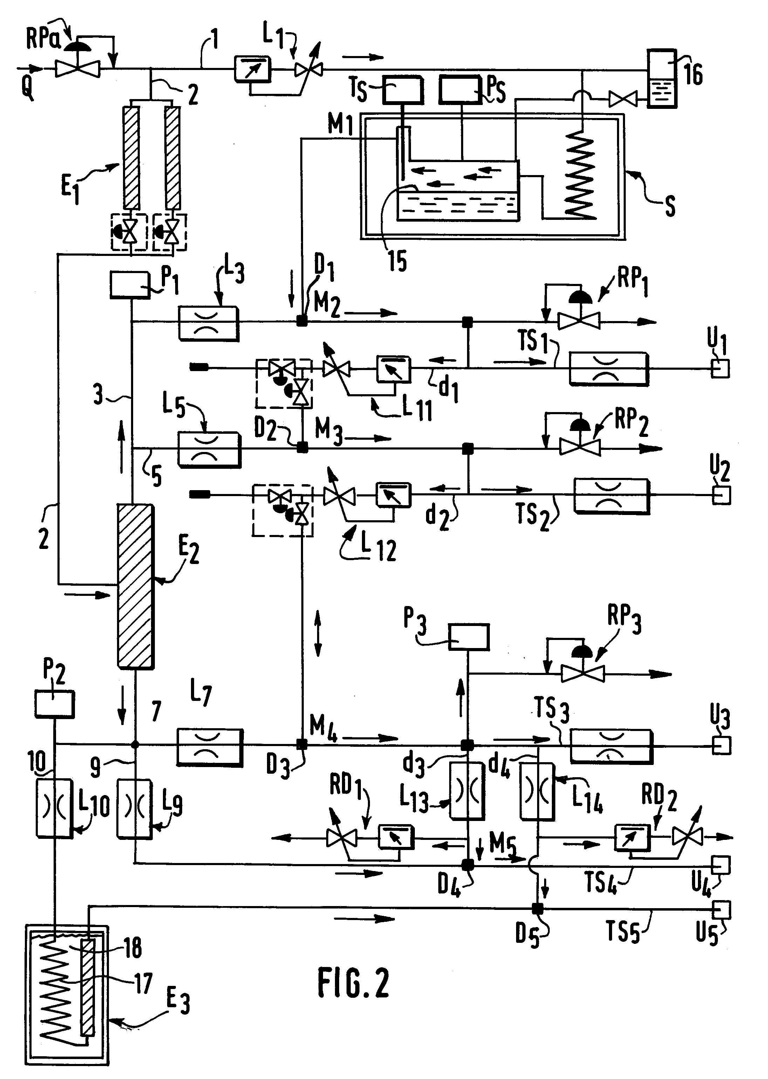

On a représenté plus en détail sur la figure 2 un dispositif selon l'invention dans une application pour l'étalonnage d'hygromètres. La pression d'alimentation du système en gaz vecteur est assurée, en amont des lignes 1 et 2, par un régulateur de pression amont $RP_a$. Dans ce mode de réalisation, le gaz à analyser A étant de la vapeur d'eau, le dispositif d'introduction S est ici constitué d'un saturateur thermostaté à double enveloppe ménageant sur le trajet du gaz dans la première ligne 1 une surface plane d'eau 15, la masse d'eau étant entretenue par un réservoir d'eau 16, la température et la pression du ciel gazeux au-dessus de la nappe d'eau 15 étant régulées à une pression $P_S$ et une température $T_S$ de saturation. A cet effet, le moyen limiteur de débit $L_1$ dans la ligne 1 est ici constitué d'un régulateur de débit assurant, en sortie du saturateur S, un débit constant du mélange initial de gaz humide $M_1$, qui sera dilué successivement par le gaz vecteur sec aux postes de dilution $D_1$ à $D_5$. Dans ce mode de réalisation, la dixième ligne 10 comporte un épurateur $E_3$ constitué d'un piège cryogénique avec un échangeur de chaleur 17 dans un bain d'azote liquide 18 et la dixième ligne 10 se raccorde, en un cinquième poste de dilution $D_5$, à une quatrième dérivation $d_4$ provenant, comme la troisième dérivation $d_3$, du troisième tronçon de sortie $TS_3$ relié à la troisième sortie $U_3$, la quatrième dérivation $d_4$ étant pour-

vue d'un moyen limiteur de débit $L_{14}$. Typiquement, les moyens limiteurs de débit $L_3$, $L_5$, $L_7$, $L_9$, $L_{10}$, $L_{13}$ et $L_{14}$ sont constitués de restricteurs fixes à col sonique tandis que les moyens limiteurs de débit $L_{11}$ dans la première dérivation $d_1$ et $L_{12}$ dans la deuxième dérivation $d_2$ sont constituées de régulateurs de débit commandables.

Avantageusement, selon un aspect de l'invention, en amont des restrictions $L_{13}$ et $L_{14}$ dans les dérivations $d_3$ et $d_4$, pour maintenir en balayage permanent toutes les lignes du système et assurer une régulation des débits précise et peu susceptible de dérives dans le temps, et donc de garantir une qualité d'analyse particulièrement fiable, les tronçons de sortie $TS_1$ à $TS_3$ sont associés à des régulateurs de pression $RP_1$, $RP_2$ et $RP_3$, respectivement disposés dans des lignes de charge de flux circulatoire en excès débouchant à l'air libre ou dans une capacité de récupération de mélange en excès, tandis que les quatrième et cinquième tronçons de sortie $TS_4$ et $TS_5$ sont associés chacun à un régulateur de débit de décharge $RD_1$ et $RD_2$ dans un circuit de décharge correspondant. Un agencement de ce type est décrit dans le document EP-A--0.479.633 dont le contenu est supposé intégré ici pour référence. Avantageusement, un capteur de pression $P_1$ est prévu en amont des restrictions $L_3$ et $L_5$, un capteur de pression $P_2$ est prévu en amont des restrictions $L_7$, $L_9$ et $L_{10}$ et un capteur de pression $P_3$ est prévu dans la ligne de décharge du troisième tronçon de sortie $TS_3$, en amont du régulateur de pression $RP_3$ pour détecter la pression dans le troisième tronçon de sortie $TS_3$, en amont des restrictions $L_{13}$ et $L_{14}$ dans les dérivations $d_3$ et $d_4$.

A titre d'exemple, les paramètres suivants pourront être adoptés pour le système de la figure 2 :
- le gaz vecteur est un gaz inerte sec, typiquement de l'azote ou de éventuellement l'argon ;
- le débit d'alimentation Q est de l'ordre de 15 litres/ minute et la pression amont, fixée par le régulateur de pression $RP_a$ est de $8 \times 10^5$ Pa ;
- le débit $q_1$ est régulé par le régulateur de débit $L_1$ et d'environ 1 litre/minute et, pour une température de saturation $T_S$ entre 10 et 20°C et une pression de saturation $P_S$ entre 3 et $5 \times 10^5$ Pa, la teneur en eau du mélange $M_1$ en sortie du saturateur est de l'ordre de 4000 ppm ;
- le débit $q_3$, déterminé par la restriction $L_3$, est de l'ordre de 3,5 litres/minute tandis que les débits $q_5$, $q_7$, $q_9$ et $q_{10}$, fixés par les restrictions $L_5$, $L_7$, $L_9$ et $L_{10}$, sont de l'ordre de 2,5 litres/minute, de façon à fournir aux différentes sorties $U_1$ à $U_5$

## Revendications

1. Procédé de fourniture, à au moins un analyseur ($a_i$) à haute sensibilité, de faibles quantités d'un premier gaz (A) contenu dans un second gaz, comprenant les étapes de diviser un flux primaire (Q) du deuxième gaz en un premier flux ($q_1$) et un deuxième flux ($q_2$) supérieur au premier flux, d'introduire dans le premier flux ($q_1$) une quantité déterminée du premier gaz pour constituer un premier mélange ($M_1$), et de combiner au moins une partie du deuxième flux ($q_2$) avec le premier mélange ($M_1$) pour constituer un deuxième mélange ($M_2$) disponible en une première sortie d'utilisation ($U_1$) pour fourniture à l'analyseur ($a_i$), caractérisé en ce qu'il comprend les étapes de diviser le deuxième flux ($q_2$) en un troisième flux ($q_3$), pour combinaison avec le premier mélange ($M_1$), et un quatrième flux ($q_4$) supérieur au troisième flux, de prélever une partie du deuxième mélange ($M_2$) avant fourniture à la première sortie ($U_1$) et de combiner au moins une partie du quatrième flux ($q_4$) avec la partie prélevée du deuxième mélange ($M_2$) pour constituer un troisième mélange ($M_3$) disponible en une deuxième sortie d'utilisation ($U_2$) pour fourniture à un analyseur ($a_i$).

2. Procédé selon la revendication 1, caractérisé en ce qu'il comprend l'étape de diviser le quatrième flux ($q_4$) en un cinquième flux ($q_5$), pour combinaison avec la partie prélevée du deuxième mélange ($M_2$), et un sixième flux ($q_6$) supérieur au cinquième flux, de prélever une partie du troisième mélange ($M_3$) avant fourniture à la deuxième sortie d'utilisation ($U_2$), et de combiner au moins une partie du sixième flux ($q_6$) avec la partie prélevée du troisième mélange ($M_3$) pour constituer un quatrième mélange ($M_4$) disponible en une troisième sortie d'utilisation ($U_3$) pour fourniture à un analyseur ($a_i$).

3. Procédé selon la revendication 2, caractérisé en ce qu'il comprend les étapes de diviser le sixième flux ($q_6$) en un septième flux ($q_7$), pour combinaison avec la partie prélevée du troisième mélange ($M_3$), et un huitième flux ($q_8$) supérieur au septième flux, de prélever une partie du quatrième mélange ($M_4$) avant fourniture à la troisième sortie d'utilisation ($U_3$), et de combiner au moins une partie du huitième flux avec la partie prélevée du quatrième mélange ($M_4$) pour constituer un cinquième mélange ($M_5$) disponible en une quatrième sortie d'utilisation ($U_4$) pour fourniture à un analyseur ($a_i$).

4. Procédé selon la revendication 3, caractérisé en ce qu'il comprend les étapes de diviser le huitième flux ($q_8$) en un neuvième flux ($q_9$) pour combinaison avec la partie prélevée du quatrième mélange ($M_4$), et un dixième flux ($q_{10}$), de faire passer le dixième flux dans un piège cryogénique ($E_3$) et de le combiner avec la partie prélevée du quatrième mélange ($M_4$) pour fourniture via une cinquième sortie d'utilisation (U₅), à un analyseur (aᵢ).

5.  Procédé selon l'une des revendications 1 à 4, caractérisé en ce qu'il comprend l'étape d'épurer au moins le deuxième flux (q₂).

6.  Procédé selon l'une des revendications 1 à 5, caractérisé en ce que chaque flux (q₃, q₅, q₇, q₉, q₁₀) combiné avec un mélange (M₁ à M₄) est déterminé par une restriction fixe (L₃, L₅, L₇, L₉, L₁₀).

7.  Procédé selon l'une des revendications 1 à 6, caractérisé en ce que le premier flux (q₁) est modulable (L₁).

8.  Procédé selon l'une des revendications 1 à 7, caractérisé en ce que le premier gaz (A) est de la vapeur d'eau.

9.  Procédé selon la revendication 8, caractérisé en ce que la vapeur d'eau est introduite dans le deuxième gaz par saturation du deuxième gaz par léchage avec une surface plane d'eau (15).

10. Dispositif de fourniture de gaz à au moins un analyseur (aᵢ) à haute sensibilité pour la mise en oeuvre du procédé selon l'une des revendications précédentes, comprenant une source de deuxième gaz (100) alimentant une première ligne (1) comportant un moyen (S) d'introduction du deuxième gaz (A), et une deuxième ligne (2) reliée à la première ligne (1) en aval du moyen d'introduction (S) et se terminant par un premier tronçon de sortie (TS₁) connectable à l'analyseur (aᵢ), caractérisé en ce que la deuxième ligne (2) se divise en une troisième ligne (5) reliée à une première dérivation (d₁) provenant du premier tronçon de sortie (TS₁) et se terminant en un deuxième tronçon de sortie (TS₂) connectable à l'analyseur (aᵢ).

11. Dispositif selon la revendication 10, caractérisé en ce que la deuxième ligne (2) se divise en une quatrième ligne (7) reliée à une deuxième dérivation (d₂), provenant du deuxième tronçon de sortie (TS₂), et se terminant en un troisième tronçon de sortie (TS₃) connectable à l'analyseur (aᵢ).

12. Dispositif selon la revendication 11, caractérisé en ce que la deuxième ligne se divise en une cinquième ligne (9) reliée à une troisième dérivation (d₃) provenant du troisième tronçon de sortie (TS₃) et se terminant en un quatrième tronçon de sortie (TS₄) connectable à l'analyseur (aᵢ).

13. Dispositif selon la revendication 11, caractérisé en ce que la deuxième ligne (2) se divise en une sixième ligne (10), traversant un piège cryogénique (E₃), reliée à une quatrième dérivation (d₄) provenant du troisième tronçon de sortie (TS₃), et se terminant en un cinquième tronçon de sortie (TS₅) connectable à l'analyseur (aᵢ).

14. Dispositif selon l'une des revendications 10 à 13, caractérisé en ce que la deuxième ligne (2) comprend au moins un dispositif d'épuration (E₁, E₂).

15. Dispositif selon l'une des revendications 10 à 14, caractérisé en ce que chaque ligne (3, 5, 7, 9, 10) dérivée de la deuxième ligne (2), comprend, en amont, une restriction fixe (L₃, L₅, L₇, L₉, L₁₀).

16. Dispositif selon l'une des revendications 10 à 15, caractérisé en ce que chaque ligne (3, 5, 7, 9, 10) dérivée de la deuxième ligne est associée à un dispositif (RP₁, RP₂, RP₃, RD₁, RD₂) de décharge d'excès de flux de gaz dans la ligne.

17. Dispositif selon l'une des revendications 10 à 16, où le premier gaz (A) est de la vapeur d'eau, caractérisé en ce que le moyen d'introduction (S) est un saturateur thermostaté à plan d'eau (15).

18. Dispositif selon la revendication 13 et la revendication 17, caractérisé en ce que le piège cryogénique (E₃) comprend un moyen (17) d'échange de chaleur avec un bain d'azote liquide (18).

19. Procédé de fourniture à au moins un analyseur (aᵢ) de traces d'un gaz à analyser par dilution d'un mélange de gaz (M₁) constitué d'un mélange du gaz à analyser (A) avec un gaz vecteur pour former un mélange d'analyse, caractérisé en ce qu'il comprend les étapes de réaliser, à partir du mélange de gaz (M₁), au moins un premier (M₂) et un deuxième (M₃) mélanges d'analyse différents, le deuxième mélange d'analyse (M₃) ayant une teneur en gaz à analyser inférieure à celle du premier mélange d'analyse, et étant obtenu par dilution d'une partie du premier mélange (M₂) avec le gaz vecteur, et en ce que les premier (M₂) et deuxième (M₃) mélanges d'analyse sont adressés, pour étalonnage ou calibration, à deux analyseurs différents (a₁, a₂).

FIG.1

FIG.2

Office européen
des brevets

**RAPPORT DE RECHERCHE EUROPEENNE**

Numero de la demande

EP    92 40 1595

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-0 370 151 (L'AIR LIQUIDE) | 1-7, 10-14,15 | G01N33/00 |
| A | * le document en entier * | 19 | G01N1/22 |
| | --- | | G01N30/00 |
| | | | G01N19/10 |
| X | EP-A-0 370 870 (L'AIR LIQUIDE) | 1-7, 10-14,15 | G01D18/00 |
| A | * le document en entier * | 19 | |
| | --- | | |
| A | PATENT ABSTRACTS OF JAPAN vol. 9, no. 203 (P-381)(1926) 21 Août 1985 & JP-A-60 066 142 ( SHISAKA KENKYUSHO K.K. ) 16 Avril 1985 * abrégé * | 8,9,17 | |
| | --- | | |
| A | MEASUREMENTS. vol. 1, no. 4, Octobre 1983, LONDON GB pages 172 - 176 H. UCHIYAMA, ET AL. 'THE TECHNIQUE OF PRODUCING REFERENCE-STANDARD GAS BY THE FLOW -MEASURING METHOD' * page 172, alinéa 1 - page 174, alinéa 1; figures 1,2,3 * | 1,10 | |

DOMAINES TECHNIQUES RECHERCHES (Int. Cl.5 )

G01N

-----

Le présent rapport a été établi pour toutes les revendications

| Lieu de la recherche | Date d'achèvement de la recherche | Examinateur |
|---|---|---|
| LA HAYE | 12 OCTOBRE 1992 | R.A.P. BOSMA |

EPO FORM 1503 03.82 (P0402)